# FASCICULE DE BREVET EUROPEEN

(11) **EP 0 914 806 B1**
(45) Date de publication et mention de la délivrance du brevet: **14.01.2004**
(21) Numéro de dépôt: 98402421.6
(22) Date de dépôt: 29.09.1998
(51) Int. Cl.: A61B 17/15

(54) **Dispositif de coupe fémorale pour la pose d'une prothèse totale de genou de reprise**
Gerät zur Durchtrennung des Femurs zum Einsetzen einer Kniegelenkprothese
Femur cutting apparatus for the replacement of a knee joint prothesis

(30) Priorité: 31.10.1997 FR 9713888
(43) Date de publication de la demande: 12.05.1999
(73) Titulaire: PROTEK SYNTHES S.A., 25461 Etupes Cédex (FR); Diomed, 25460 Etupes (FR)
(72) Inventeur: Aebi, Jürg, 25900 Besancon (FR); Beaufils, Philippe, 25900 Besancon (FR); Bouraly, Jean-Pierre, 25900 Besancon (FR); De Lestang, Michel, 25900 Besancon (FR); Gaffury, Jean-Gilles, 25900 Besancon (FR); Hourlier, Hervé, 25900 Besancon (FR); Lallement, Jean-Jacques, 25900 Besancon (FR); Legroux, Philippe, 25900 Besancon (FR); Levai, Jean-Paul, 25900 Besancon (FR); Pondaven, Gérard, 25900 Besancon (FR); Schuster, Pierre, 25900 Besancon (FR); Vergnat, Christian, 25900 Besancon (FR)
(74) Mandataire: Bentz, Jean-Paul

(56) Documents cités:
- WO-A-97/16129
- US-A- 4 944 760
- US-A- 5 258 032
- US-A- 5 464 406

## Description

La présente invention concerne un dispositif de coupe fémorale destiné à la pose d'une prothèse de reprise de genou.

L'invention trouve une application particulièrement avantageuse dans le domaine de l'arthroplastie totale du genou.

Une prothèse de genou est soumise à un très grand nombre de contraintes mécaniques dès que l'articulation est sollicitée. Si pour quelques raisons que ce soit, l'implantation de cette prothèse a été mal réalisée, on assiste rapidement à une résorption de l'os et conséquemment à une désolidarisation progressive des différents composants. Il devient alors indispensable de procéder au plus vite au remplacement de la totalité de l'implant par une prothèse de reprise qu'il conviendra de positionner cette fois de manière adéquate.

Habituellement, l'opération de remplacement consiste tout d'abord à dissocier délicatement la prothèse originelle de l'os, puis à l'extraire tout aussi précautionneusement de manière à éviter les fractures et les pertes osseuses inutiles. La grande difficulté qui survient ensuite est de pouvoir se repositionner correctement dans l'espace au niveau de chaque portion osseuse participant à l'articulation du genou, à savoir l'extrémité distale du fémur, la face postérieure de la rotule et l'extrémité proximale du tibia.

C'est au niveau du fémur que ce type de problème est le plus délicat. A ce stade de l'opération, la partie distale de cet os est en effet dépourvue de véritables repères anatomiques consécutivement aux coupes réalisées lors de l'implantation du composant fémoral originel, aux résorptions qui se sont produites ensuite et aux éventuelles pertes osseuses survenues lors du retrait dudit composant fémoral.

La présence de nombreux défauts osseux à la partie distale du fémur impose alors de procéder à de nouvelles coupes. Celles-ci sont généralement réalisées de manière tout à fait conventionnelle au moyens de boîtiers et/ou de guides de coupe stabilisés axialement par rapport à l'os. Cependant, en l'absence de points de repère significatifs, ces coupes s'effectuent sans considérations dimensionnelles particulières, c'est-à-dire sans prendre véritablement en compte au préalable la conformation interne du futur implant et/ou son positionnement relatif spécifique par rapport au tibia. L'essentiel est simplement d'obtenir des surfaces d'appui planes et saines afin de garantir ultérieurement un positionnement stable de la prothèse de reprise. Ce n'est qu'à posteriori que l'on va tenter de déterminer la forme adéquate du composant fémoral. Pour cela, on utilise un composant fémoral d'essai sur lequel on rajoute des cales de hauteurs variables. On procède alors par tâtonnement jusqu'à ce que les pertes osseuses soient compensées au maximum, tout en veillant à l'aide d'entretoises à ce que l'intervalle entre l'extrémité distale du fémur et l'extrémité proximale du tibia soit similaire en flexion et en extension (voir par exemple US-A-5 464 406).

Ce type de technique opératoire présente l'inconvénient d'être fondamentalement d'une très grande imprécision puisque l'évaluation de la forme et du positionnement du futur implant ne s'effectue qu'après avoir réalisé les différentes coupes à l'extrémité du fémur. Dans ces conditions, il ne faut bien évidemment pas s'étonner que l'étape de coupe s'accomplisse sans point de repère. De même, il n'est pas possible que l'étape d'évaluation précitée soit exécutée autrement que par tâtonnement.

Cet état de fait regrettable résulte essentiellement de la simplicité conceptuelle des dispositifs de coupe fémorale communément utilisés. Leur structure se limite en effet à de simples boîtiers et/ou guides de coupe que l'on vient stabiliser successivement à la partie distale du fémur à l'aide de moyens de centrage axiaux. Leur fonction est tout aussi restreinte puisqu'elle se borne à réaliser des surfaces planes et saines en prenant comme points de repère les différents défauts osseux présents à l'extrémité de l'os. Les coupes à réaliser sont classiquement les coupes distale, postérieure et antérieure. De plus, l'encombrement général de ces dispositifs de coupe impose l'immobilisation totale du genou et entraîne par conséquent l'impossibilité de procéder à des simulations de mouvement en flexion/extension.

Aussi, le problème technique à résoudre par l'objet de la présente invention est de proposer un dispositif de coupe fémorale pour la pose d'une prothèse totale de genou de reprise en chirurgie osseuse, comportant des moyens de centrage à l'extrémité distale du fémur ainsi que des moyens de coupe de la partie distale de l'os, dispositif de coupe fémorale qui permettrait de réaliser des coupes à posteriori, c'est-à-dire après avoir déterminé les dimensions et le positionnement dans l'espace du futur composant fémoral.

La solution au problème technique posé consiste, selon la présente invention, en ce que les moyens de coupe sont solidaires d'un organe de positionnement monté mobile en déplacement par rapport aux moyens de centrage, au moins une partie de l'organe de positionnement présentant une forme externe apte à coopérer par glissement avec un composant tibial implanté à l'extrémité proximale du tibia correspondant, quelle que soit la position relative dudit organe de positionnement par rapport audit composant tibial au cours d'un mouvement de flexion/extension du genou.

L'invention telle qu'ainsi définie présente l'avantage de permettre un positionnement des moyens de coupe en fonction du tibia et non en fonction du fémur. Contrairement aux dispositifs de l'état de la technique, les moyens de coupe ne sont en effet plus fixés en appui contre l'extrémité distale du fémur, mais positionnés dans l'espace au contact d'un composant tibial préalablement implanté à l'extrémité proximale du tibia. Le composant tibial utilisé peut être avantageusement une embase d'essai.

La combinaison de la mobilité de l'organe de positionnement, avec la forme particulière de sa face externe, permet de définir une position apte à garantir le contact entre ledit organe de positionnement et le composant fémoral, quelle que soit leur position relative.

Le fait que l'organe de positionnement puisse coopérer par glissement avec le composant tibial permet de simuler de manière continue un quelconque mouvement de flexion/extension du genou. Cela permet avantageusement de valider les choix concernant la taille, la forme et/ou le positionnement du futur implant fémoral avant la réalisation proprement dite des différentes coupes.

Selon une particularité de l'invention, la forme externe de l'organe de positionnement est sensiblement identique au moins à la forme externe des parties postérieure et distale du composant fémoral à implanter. Ainsi, une fois correctement positionné dans l'espace à l'extrémité distale du fémur, l'organe de positionnement bénéficie d'un comportement conforme à celui qu'aurait le composant fémoral si ce dernier était implanté de manière optimale. Il est par conséquent possible de procéder à toute une série de contrôles et de tests afin de valider avec précision la position et les caractéristiques dimensionnelles du futur composant fémoral. Une fois cette opération terminée, les différentes coupes sont réalisées conformément aux choix précédemment arrêtés, c'est-à-dire en fonction de la position de l'organe de positionnement et de la conformation globale du composant fémoral à implanter.

De manière particulièrement avantageuse, la forme externe de l'organe de positionnement présente des caractéristiques formelles et dimensionnelles sensiblement identiques à celles qui sont communes aux formes externes des différentes tailles du composant fémoral pouvant être utilisées. Ainsi conformé et après un positionnement adéquat, l'organe de positionnement présente un comportement similaire à celui du composant fémoral, quelle que soit la taille de ce dernier. Par conséquent le dispositif de coupe fémorale selon l'invention peut être avantageusement utilisé pour la pose de prothèses de reprise structurellement identiques mais de format variable. Les caractéristiques communes aux différentes tailles du composant fémoral concernent essentiellement les formes et les dimensions des parties distales et postérieures, et notamment celles correspondant aux condyles.

Selon une autre particularité de l'invention, l'organe de positionnement est monté mobile en translation transversale par rapport aux moyens de centrage, suivant une direction orthogonale à l'axe médullaire de l'os. Cette mobilité transversale permet de positionner l'organe de positionnement suivant un axe antéro-postérieur, en fonction de la taille du composant fémoral que l'on envisage d'implanter.

Selon une autre particularité de l'invention, l'organe de positionnement est monté mobile en translation axiale par rapport aux moyens de centrage, suivant une direction parallèle à l'axe médullaire de l'os. La mobilité axiale ainsi obtenue permet ici de positionner l'organe de positionnement en écartement distal par rapport à l'os, en fonction de la position du plateau tibial.

De manière particulièrement avantageuse, l'organe de positionnement est monté mobile en rotation axiale par rapport aux moyens de centrage, autour d'une direction parallèle à l'axe médullaire de l'os. L'organe de positionnement peut ainsi être correctement positionné en rotation axiale à l'extrémité distale du fémur en fonction de repères anatomiques tels que l'axe antéro-postérieur, la ligne bicondylienne et/ou l'axe mécanique du tibia.

De préférence, l'organe de positionnement est monté mobile en rotation transversale par rapport aux moyens de centrage, autour d'une direction orthogonale à l'axe médullaire du fémur. La finalité de cette mobilité est de permettre, d'une part, l'adaptation du dispositif de coupe fémorale selon l'invention indifféremment sur un genou droit ou sur un genou gauche, et d'autre part, la prise en compte de l'angle séparant l'axe anatomique et l'axe mécanique de l'os.

La description qui va suivre en regard des dessins annexés, donnés à titre d'exemples non limitatifs, fera bien comprendre en quoi consiste l'invention et comment elle peut être réalisée.

La figure 1 est une vue en perspective d'un dispositif de coupe fémorale conforme à l'invention.

La figure 2 constitue une vue de la face interne du dispositif illustré à la figure 1, montrant notamment la mobilité de l'organe de positionnement en translation transversale et en rotation orthogonale à l'axe de l'os.

La figure 3 est une vue en perspective du dispositif de coupe fémorale de la figure 1, sur lequel sont montés des moyens de calibrage aptes à repérer la position de l'organe de positionnement suivant l'axe antéro-postérieur, en fonction de la taille du composant fémoral que l'on désire implanter.

La figure 4 représente en perspective le dispositif de coupe fémorale de la figure 1, sur lequel sont montés des moyens de coupe distale ainsi que des moyens de blocage aptes à immobiliser l'organe de positionnement en translation axiale.

La figure 5 est une vue de dessus des moyens de coupe distale et des moyens de blocage illustrés à la figure 4.

La figure 6 représente schématiquement en coupe longitudinale, un mécanisme de réglage de la longueur des moyens de blocage.

La figure 7 constitue une vue en perspective du dispositif de coupe fémorale de la figure 1, sur lequel sont montés des moyens de visée apte à déterminer la position en rotation axiale de l'organe de positionnement par rapport au fémur.

La figure 8 est une vue en perspective du dispositif de coupe fémorale de la figure 1, pourvu des moyens de coupe antérieure conformes à l'invention.

Pour des raisons de clarté, les mêmes éléments ont été désignés par des références identiques. De même, seuls les éléments essentiels pour la compréhension de l'invention ont été représentés, et ceci sans respect de l'échelle et de manière schématique.

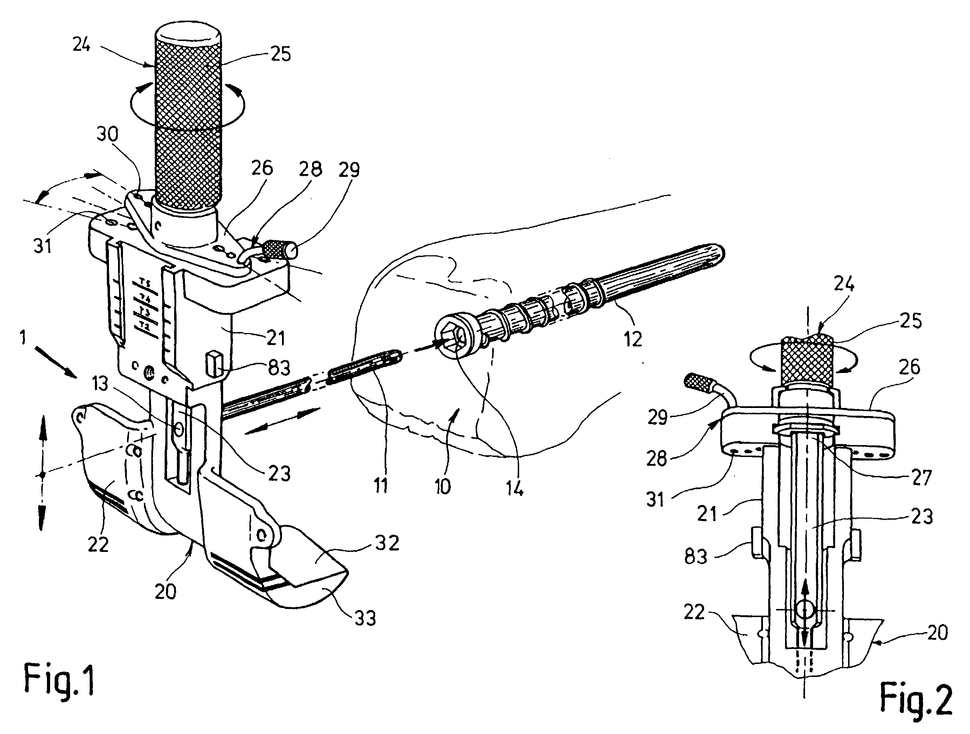

Dans le mode de réalisation particulier illustré sur les figures 1 et 2, le dispositif de coupe fémorale 1 comporte notamment des moyens de centrage 10 par rapport auxquels est monté mobile en déplacement un organe de positionnement 20 destiné à supporter les différents moyens de coupe nécessaires à la redéfinition formelle de la partie distale du fémur.

L'organe de positionnement 20 se compose schématiquement d'un corps principal 21 et d'une partie active 22 formant surface d'appui afin de pouvoir coopérer par glissement avec un composant tibial implanté dans le tibia. A cet effet, la forme externe de la partie active 22 est sensiblement identique à celle des parties respectivement distale et postérieure du composant fémoral définitif que l'on envisage de mettre en place à l'extrémité du fémur.

Dans cet exemple de réalisation, les moyens de centrage 10 comportent une tige de guidage 11 apte à coopérer par coulissement avec une tige filetée 12 formant tire-fond. La tige de guidage 11 et la tige filetée 12 sont destinées respectivement à être solidarisée à l'organe de positionnement 20 et à être implantée à l'intérieur du canal médullaire du fémur. Bien entendu, la tige filetée 12 peut être remplacée par n'importe quel organe intramédullaire susceptible de remplir les mêmes fonctions d'ancrage et de centrage. Les tire-fonds sont tout à fait indiqués lorsque le fémur présente suffisamment de tissu spongieux dans sa partie épiphysaire. Dans le cas contraire, un système différent est préféré pour obtenir un point d'appui efficace. L'organe intramédullaire comporte alors une tige centreuse de grande longueur, aux extrémités de laquelle sont solidarisés un centreur et un cône de blocage dont les diamètres respectifs peuvent être choisis parmi plusieurs tailles. L'ensemble de ce dispositif peut être avantageusement impacté ou retiré à l'aide d'un impacteur extracteur universel.

L'organe de positionnement 20 présente de nombreuses mobilités par rapport aux moyens de centrage 10, tant en translation qu'en rotation. L'une d'entre elles concerne plus particulièrement une translation transversale selon une direction orthogonale à l'axe médullaire de l'os, c'est-à-dire dans cet exemple de réalisation selon une direction orthogonale à l'axe suivant lequel sont positionnés les moyens de centrage 10.

Ce mouvement est autorisé grâce au fait que l'extrémité externe 13 de la tige de guidage 11 est solidarisé perpendiculairement à un axe 23 de section carrée, monté longitudinalement coulissant dans le corps 21 de l'organe de positionnement 20. Le déplacement proprement dit de l'axe 23 est généré par des moyens de réglage 24, comportant dans cet exemple de réalisation une molette 25 montée mobile en rotation par rapport à une pièce de guidage 26 solidaire de l'organe de positionnement 20, la pièce de guidage 26 étant chargée notamment d'empêcher toute rotation dudit axe 23.

La transformation du mouvement de rotation animant la molette 25 en un mouvement de translation transmissible à l'axe 23 est permise grâce au fait que ladite molette 25 est pourvue d'un alésage central fileté dans lequel vient coopérer par vissage l'extrémité supérieure 27 dudit axe 23. L'alésage ménagé au centre de la pièce de guidage 26 présente une section carrée complémentaire de celle de l'axe 23, de sorte que la mise en rotation dudit axe 23 est évitée lors de l'actionnement de la molette 25 et qu'en contre-partie le vissage devient effectif au niveau des moyens de réglage 24. L'axe 23 va alors être entraîné en translation, ce qui va avoir pour conséquence de mobiliser l'organe de positionnement 20 par rapport aux moyens de centrage 10. Grâce à leur structure particulière, les moyens de réglage 24 sont également aptes à assurer une immobilisation de l'organe de positionnement 20 par rapport aux moyens de centrage 10.

Afin de pouvoir prendre en compte l'écart angulaire existant entre l'axe anatomique et l'axe mécanique du fémur, l'organe de positionnement 20 présente avantageusement une mobilité en rotation transversale selon une direction orthogonale à l'axe médullaire de l'os, c'est-à-dire ici selon une direction orthogonale à l'axe suivant lequel sont positionnés les moyens de centrage 10.

Dans cet exemple de réalisation, l'axe 23 est à priori bloqué en rotation par la pièce de guidage 26. Seule une mise en rotation de cette dernière peut permettre à l'organe de positionnement 20 de pivoter transversalement par rapport aux moyens de centrage 10. Aussi, la pièce de guidage 26 est montée mobile en rotation sur le corps 21 suivant une direction orthogonale à la tige de guidage 11.

Le basculement de l'organe de positionnement 20 ainsi engendré est contrôlé angulairement par des moyens d'indexage 28 comportant une clavette de verrouillage 29 et deux séries d'alésages traversants 30 et 31, ménagés en vis-à-vis à travers respectivement la pièce de guidage 26 et le corps 21. Ainsi, en engageant la clavette 29 successivement dans un alésage 30 puis un alésage 31, il est possible de bloquer la rotation de la pièce de guidage 26 par rapport au corps 21, et par conséquent celle de l'organe de positionnement 20 par rapport aux moyens de centrage 10.

Les alésages traversants 30, 31 sont ménagés de telle sorte que l'angle de rotation soit limité à des valeurs prédéterminées, correspondant à des écarts angulaires typiques entre l'axe anatomique et l'axe mécanique du fémur, c'est-à-dire 5, 7 ou 9°. Dans cette hypothèse, l'axe anatomique de l'os correspond à la direction suivant laquelle sont disposés les moyens de centrage 10. De manière particulièrement avantageuse, ce type de réglage peut être réalisé de part et d'autre du plan de symétrie s'étendant orthogonalement au corps 21 de l'organe de positionnement 20, afin de pouvoir adapter le dispositif de coupe fémorale 1 aussi bien sur un genou droit que sur un genou gauche.

La tige de guidage 11 est montée librement coulissante dans un alésage longitudinal 14 ménagé au centre de la tige filetée 12 formant tire-fond. L'organe de positionnement 20, auquel ladite tige de guidage 11 est solidarisée par vissage, se voit par conséquent pourvu d'une double mobilité en translation et en rotation axiales selon une direction parallèle à l'axe médullaire de l'os, c'est-à-dire dans le cas présent selon une direction parallèle à l'axe suivant lequel sont positionnés les moyens de centrage 10.

Toutes les mobilités dont est doté l'organe de positionnement 20 sont destinées à permettre une mise en place optimale du dispositif de coupe fémorale 1 par rapport à la partie distale du fémur. Cette opération consiste à positionner précisément la partie active 22 de l'organe de positionnement 20 à l'endroit exact que devrait occuper les parties correspondantes du composant fémoral définitif pour obtenir une stabilité parfaite aussi bien en flexion qu'en extension, ainsi que pour toutes les valeurs angulaires intermédiaires.

Pour atteindre un positionnement idéal de l'organe de positionnement 20, il est nécessaire de déterminer essentiellement trois composantes, à savoir l'écartement en distal, la position suivant l'axe antéro-postérieur et la position en rotation par rapport à l'axe médullaire de l'os. A cette fin, on utilise différents systèmes de repérage dans l'espace combinés aux mobilités respectivement en translation axiale, en translation transversale et en rotation axiale.

Le positionnement de l'organe de positionnement 20 suivant l'axe antéro-postérieur, c'est-à-dire suivant une direction perpendiculaire à l'axe médullaire de l'os mais sensiblement parallèle au plan sagittal, s'effectue en fonction de la taille du futur composant fémoral que l'on envisage d'implanter. Afin de prendre en compte ce paramètre, on utilise des moyens de calibrage 40 aptes à repérer la position théorique de la face interne antérieure de n'importe quelle taille de composant fémoral, par rapport à celle de la face interne postérieure de ce même composant. Dans le cas présent, la face interne postérieure correspond à la surface interne 32 de la portion postérieure 33 de la partie active 22 de l'organe de positionnement 20.

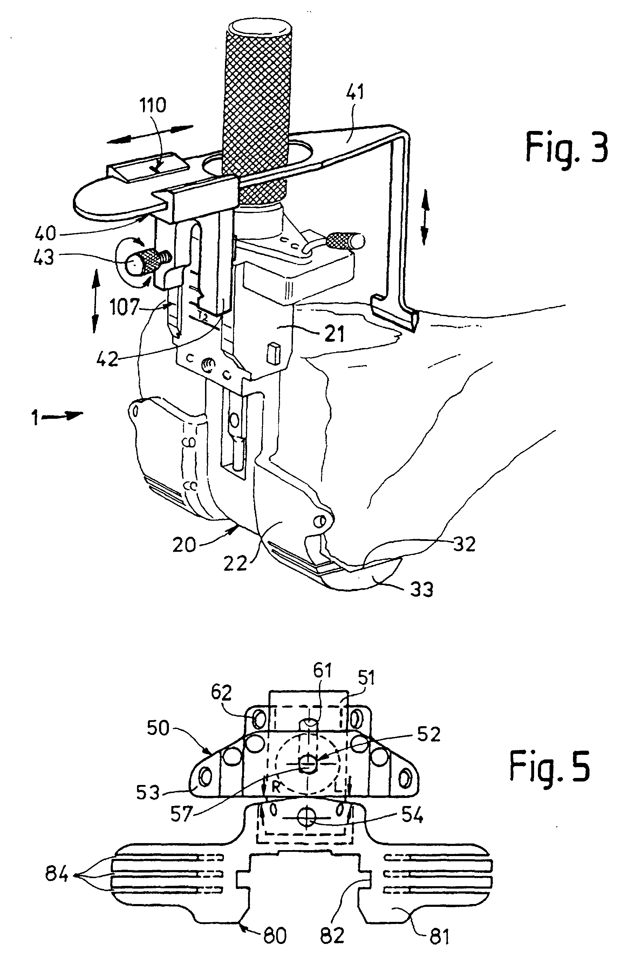

Ainsi qu'on peut le voir sur la figure 3, les moyens de calibrage 40 sont amovibles et comportent un stylet 41 monté mobile en coulissement sur une pièce support 42, elle-même montée mobile en translation antéro-postérieure sur le corps 21 de l'organe de positionnement 20. Le déplacement du stylet 41 par rapport à la pièce support 42 s'effectue par ailleurs orthogonalement à celui de ladite pièce support 42 par rapport au corps 21. Cependant, dans le cas du stylet 41 comme dans celui de la pièce support 42, la mobilité est contrôlée par des moyens de blocage constitués respectivement par un système de poussoir à bille non représenté et par une vis moletée 43. Le but est bien évidemment de permettre une immobilisation du stylet 41 et de la pièce support 42 en fonction de la taille du composant retenue. Pour cela une série de repères 107, 110 correspondant à chaque taille sont ménagés respectivement sur le corps 21 et à la surface du stylet 41, à l'endroit précis où ont lieu les coulissements. La position de la pièce support 42 par rapport au corps 21 détermine l'intervalle entre les faces internes respectives des parties antérieure et postérieure de l'implant fémoral définitif. La position du stylet 41 par rapport à ladite pièce support 42 tient quant à elle compte de la hauteur de la seule partie antérieure du futur composant.

En ce qui concerne l'écartement en distal de l'organe de positionnement 20 par rapport à l'extrémité du fémur, la détermination de la position optimale s'effectue simplement en mettant en extension le genou, puis en utilisant la mobilité en translation axiale dudit organe de positionnement 20 pour mettre en contact la partie active 22 avec un composant tibial préalablement mis en place à l'extrémité proximale du tibia.

Afin d'immobiliser le dispositif de coupe fémorale 1 à l'extrémité distale du fémur, il est avantageusement prévu des moyens de blocage 50 aptes à verrouiller la mobilité en translation axiale de l'organe de positionnement 20 par rapport aux moyens de centrage 10. Dans cet exemple particulier de réalisation, le verrouillage s'effectue de manière externe puisque les moyens de blocage 50 sont à la fois solidaires du dispositif de coupe fémorale 1 et de l'os.

Il est important de pouvoir déterminer avec précision l'intervalle qui va se former entre l'organe de positionnement 20 et l'extrémité distale du fémur, lorsque le dispositif de coupe fémorale 1 va être déplacé en translation axiale vers le tibia. En effet, c'est en fonction de cet écartement que vont être choisies les cales distales destinées à être solidarisées à l'intérieur du composant fémorale ; la hauteur de l'espace ainsi libéré devant correspondre à l'épaisseur de chaque cale utilisée. Aussi, les moyens de blocage 50 sont conçus pour être réglables en longueur ; l'allongement s'effectuant avantageusement par pas de longueur déterminée, par exemple de 4 mm.

Afin de garantir un bon fonctionnement des moyens de blocage 50, il est nécessaire que l'allongement de ceux-ci puisse être réalisé suivant une direction parallèle à l'axe le long duquel s'effectue la translation de l'organe de positionnement 20. C'est pourquoi les moyens de blocage 50 sont montés pivotants par rapport au dispositif de coupe fémorale 1, autour d'une direction sensiblement perpendiculaire à l'axe médullaire du fémur. Ainsi, ils peuvent être orientés suivant la nature droite ou gauche du genou à traiter et en fonction de l'écart angulaire existant entre l'axe anatomique et l'axe mécanique de l'os.

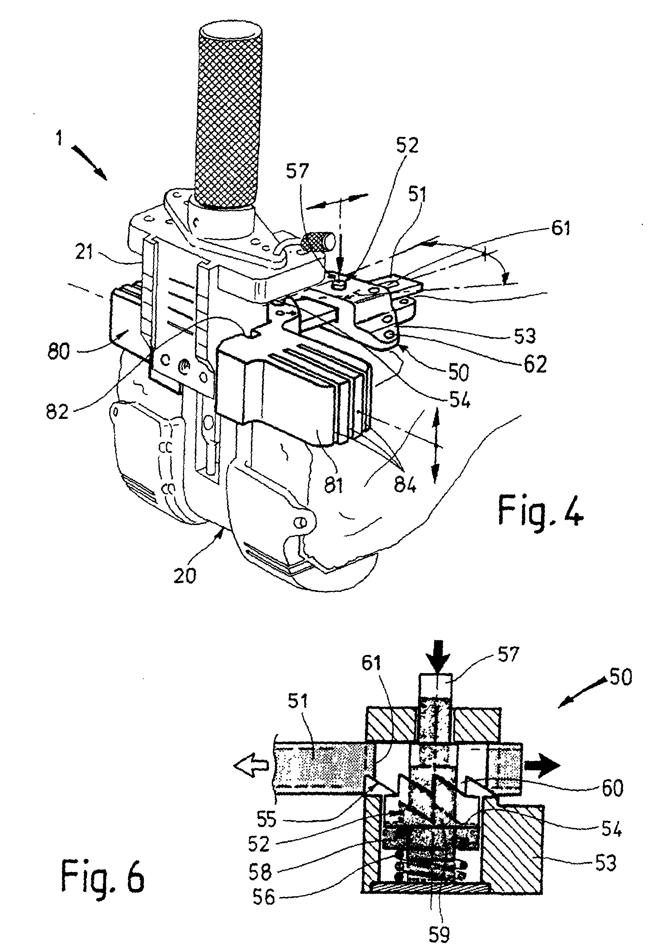

Dans le mode de réalisation particulier illustré aux figures 4 à 6, les moyens de blocage 50 sont constitués par une crémaillère 51 avec laquelle coopère par blocage un organe de verrouillage 52 disposé dans une tête d'ancrage 53. La crémaillère 51 est montée pivotante, via un axe 54, par rapport à un élément du dispositif de coupe 1 qui est ici constitué par des moyens de coupe distale 80 aptes à être fixés de manière amovible sur le corps 21 de l'organe de positionnement 20. La tête d'ancrage 53 est quant à elle destinée à être solidarisée à l'os à l'aide de broches introduites dans les nombreux alésages traversants 62 ménagés sur toute sa surface.

Ainsi qu'on peut le voir très précisément sur la figure 6, l'organe de verrouillage 52 est constitué par une pièce cylindrique 58 dont la face supérieure 54 présente une forme sensiblement complémentaire de la zone dentée 55 de la crémaillère 51. Comme l'organe de verrouillage 52 est de plus monté mobile en translation transversale par rapport à la crémaillère 51, la face supérieure 54 est en mesure de coopérer par blocage avec la zone dentée 55 ; le contact étant garanti par un ressort de compression 56.

Le déverrouillage s'effectue quant à lui en exerçant une pression sur un poussoir 57 solidaire de la pièce cylindrique 58. Cette action a pour conséquence de comprimer le ressort 56 et ainsi de désengager les crans 59 de la face supérieure 54, des crans 60 de la zone dentée 55. Il est à noter que le poussoir 57 passe à travers la crémaillère 51 grâce à la présence d'une lumière oblongue 61 ménagée longitudinalement au niveau de la zone dentée 55.

Chacun des différents crans 59, 60 présente par ailleurs un profil triangulaire composé d'un côté orthogonal à la direction de déplacement et d'un côté biseauté, disposés respectivement dans le sens de réduction et dans le sens d'allongement des moyens de blocage 50. Cette particularité permet, d'une part, d'interdire toute diminution de longueur sans avoir recours à l'organe de verrouillage 52, et d'autre part, d'autoriser l'augmentation de longueur par simple traction de la tête d'ancrage 53 par rapport à l'élément auquel est solidarisée la crémaillère 51.

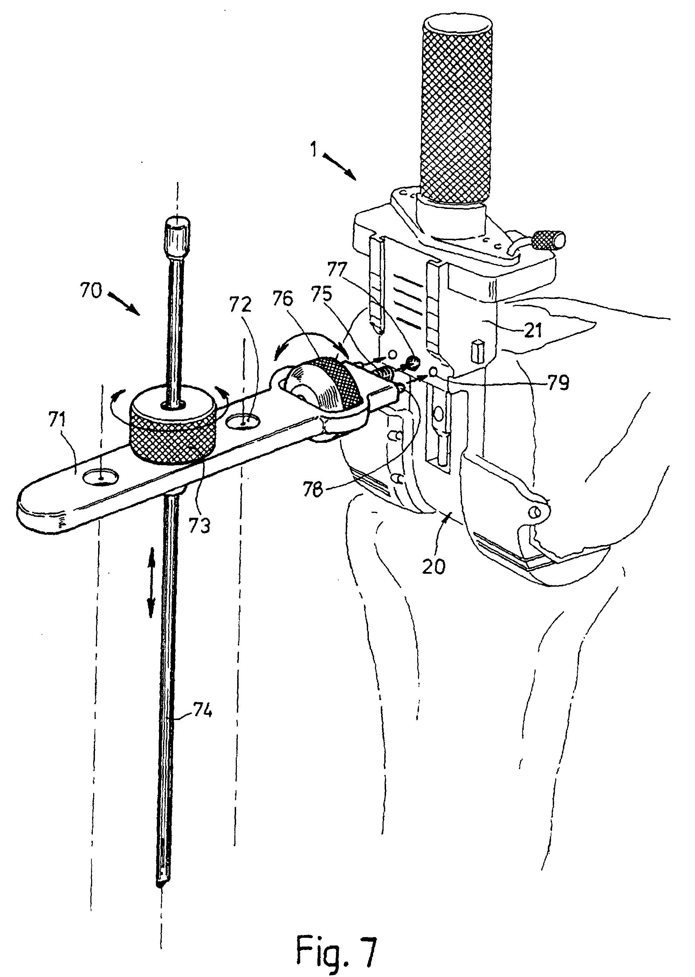

La position du dispositif de coupe fémorale 1, en rotation axiale par rapport aux moyens de centrage 10, est déterminée en utilisant des moyens de visée 70 extramédullaires. Conformément à l'exemple de la figure 7, ces moyens de visée 70 sont constitués par une poignée 71 pourvue d'une série d'alésages traversants 72, destinés à recevoir une pièce de blocage 73 dans laquelle est montée coulissante une tige de contrôle 74.

Afin de rendre les moyens de visée amovibles, la poignée 71 est dotée à l'une de ses extrémités d'une vis de fixation 75 actionnée par une molette 76, ladite vis de fixation 75 étant apte à coopérer avec un alésage taraudé 77 ménagé sur la face externe du corps 21 de l'organe de positionnement 20. De part et d'autre de la vis de fixation 75, il est avantageusement prévu deux picots 78 destinés à être introduits respectivement dans deux alésages 79 de formes complémentaires, ménagés de part et d'autre de l'alésage taraudé 77. Leur présence permet d'empêcher la mise en rotation de la poignée 71 autour de l'axe de vissage, et ainsi de garantir le parfait positionnement de la tige de contrôle 74 dans une direction parallèle à l'axe antéro-postérieur, c'est-à-dire dans le plan sagittal de l'organe de positionnement 20. Une fois la poignée 71 solidarisée, la tige de contrôle est pointée sur un repère anatomique tel que le deuxième métatarsien par exemple.

Le dispositif de coupe fémorale 1 comporte classiquement des moyens de coupe distale 80, des moyens de coupe postérieure 90, et des moyens de coupe antérieure 100.

Ainsi qu'on peut le voir dans le mode de réalisation particulier illustré aux figures 4 et 5, les moyens de coupe distale 80 sont constitués par un gabarit amovible 81 apte à être monté de manière réversible sur l'organe de positionnement 20. Pour cela, le gabarit de coupe distale 81 est pourvu de moyens de fixation, constitués dans cet exemple par des rainures 82 destinées à coopérer par coulissement avec des languettes 83 ménagées longitudinalement de part et d'autre du corps 21 de l'organe de positionnement 20.

La direction d'engagement de ces moyens de fixation de sections complémentaires est perpendiculaire à l'axe mécanique de l'os afin de garantir une stabilité parfaite des moyens de coupe distale 80 suivant cet axe. L'immobilisation du gabarit 81 suivant la direction d'engagement parallèle au plan de coupe distale s'effectue quant à elle par ancrage sur la corticale antérieure du fémur, avantageusement à l'aide des moyens de blocage 50. En effet, selon une particularité de l'invention déjà évoquée, lesdits moyens de blocage 50 sont solidaires des moyens de coupe distale 80 grâce au fait que la crémaillère 51 est montée pivotante sur le gabarit 81. Aussi, après fixation de la tête d'ancrage 53 le long de l'os, les moyens de coupe distale 80 forment un ensemble monobloc avec l'organe de positionnement 20.

Le gabarit 81 est pourvu d'une série de fentes latérales 84 définissant trois plans de coupe distale parallèles. Chacun d'entre eux correspond avantageusement à une longueur prédéterminée des moyens de réglage 50, de sorte qu'il est possible de réaliser une coupe distale identique quel que soit l'allongement desdits moyens de réglage 50, et par conséquent quel que soit l'écartement de l'organe de positionnement 20 par rapport à l'extrémité de l'os. Conformément à l'exemple choisi, si chaque cran du système de réglage en longueur des moyens de blocage 50 correspond à un allongement de 4 mm, alors l'intervalle entre les différents plans de coupe sera avantageusement choisi égal à 4 mm. L'existence de ces trois plans de coupe permet également de réaliser éventuellement des coupes différenciées d'une valeur de 4 ou 8 mm.

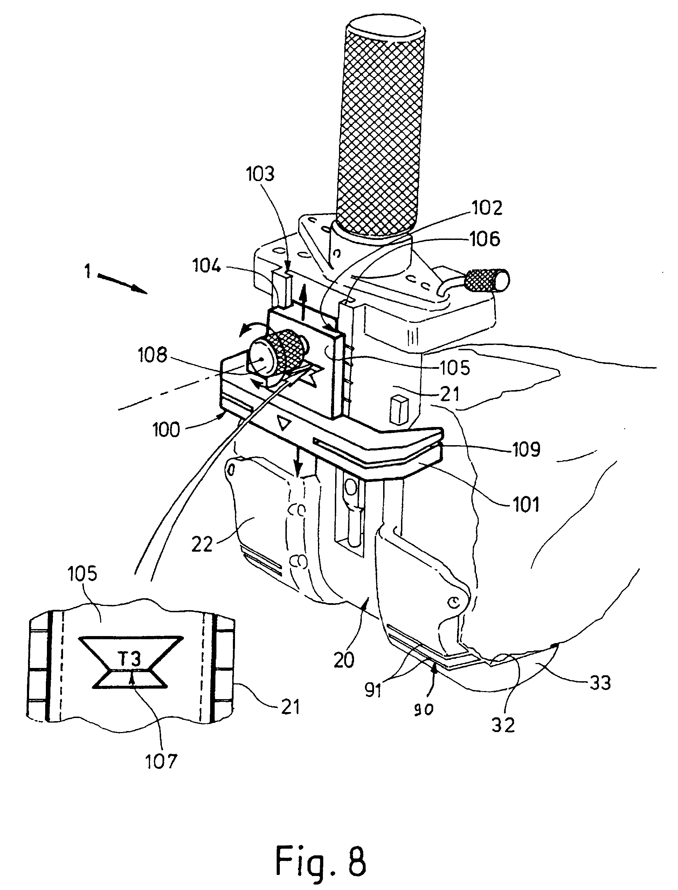

Les moyens de coupe postérieure 90 permettent eux aussi de réaliser des coupes différenciées à l'extrémité du fémur. A cet effet, il est prévu deux plans de coupe postérieure qui sont matérialisés par deux séries de fentes latérales 91 parfaitement parallèles (figure 8). Ces dernières sont ménagées directement à travers l'organe de positionnement 20, parallèlement à la surface interne 32 de la portion postérieure 33 faisant partie de la partie active 22 de l'organe de positionnement 20.

Les moyens de coupe distale 80 et les moyens de coupe postérieure 90 offrent tous deux la possibilité d'effectuer des coupes différenciées à +4 ou +8 mm. De manière particulièrement avantageuse, ces valeurs données à titre d'exemple correspondent aux épaisseurs des différentes cales de comblement disponibles pour compenser les pertes osseuses en parties distales et postérieures ; lesdites cales venant se solidariser sur les surfaces internes respectivement distales et postérieures du composant fémoral définitif.

Enfin, le dispositif de coupe fémorale 1 est pourvu de moyens de coupe antérieure 100 montés mobiles en translation sur l'organe de positionnement 20, parallèlement à la direction suivant laquelle ledit organe de positionnement 20 est monté mobile en translation transversale par rapport aux moyens de centrage 10. Le but est de pouvoir faire varier l'écartement du plan de coupe antérieure par rapport à l'axe mécanique du fémur, en fonction de la taille du composant fémoral à implanter.

Les moyens de coupe antérieure 100 sont ici constitués par un gabarit amovible 101, doté de moyens de guidage 102 aptes à coopérer par coulissement avec des moyens de guidage 103 de formes sensiblement complémentaires, ménagés sur le corps 21 de l'organe de positionnement 20. La direction de déplacement du gabarit 101 est bien entendu parallèle à l'axe antéro-postérieur.

Conformément à l'exemple de la figure 8, les moyens de guidage 102, se composent de deux rainures 104 ménagées de part et d'autre du corps central 105 du gabarit de coupe antérieure 101. De manière particulièrement avantageuse, les moyens de guidage 103 sont quant à eux constitués par les languettes 106 utilisées précédemment pour contrôler le déplacement en translation antéro-postérieure des moyens de calibrage 40. Cela permet de réemployer tout aussi judicieusement le marquage 107 visible sur les figures 1, 3, 4 et 7, afin de pouvoir repérer la position de l'élément coulissant utilisé en fonction des différentes tailles de composantes fémoraux disponibles.

Comme dans le cas des moyens de calibrage 40, une vis moletée 108 est prévue pour immobiliser le déplacement du gabarit 101 par rapport à l'organe de positionnement 20, et par conséquent pour stabiliser de manière optimale le plan de coupe antérieure unique passant par les fentes latérales 109.

Naturellement, l'invention n'est en rien limitée par les spécificités qui ont été décrites dans ce qui précède, ni par les détails des modes de réalisation particuliers choisis pour illustrer ladite invention.

## Revendications

1. Dispositif de coupe fémorale (1) pour la pose d'une prothèse totale de genou de reprise en chirurgie osseuse, ledit dispositif de coupe (1) comportant des moyens de centrage (10) à l'extrémité distale du fémur ainsi que des moyens de coupe (80, 90, 100) de la partie distale de l'os, **caractérisé en ce que** les moyens de coupe (80, 90, 100) sont solidaires d'un organe de positionnement (20) monté mobile en déplacement par rapport aux moyens de centrage (10), au moins une partie de l'organe de positionnement (20) présentant une forme externe apte à coopérer par glissement avec un composant tibial implanté dans le tibia, quelle que soit la position relative dudit organe de positionnement (20) par rapport audit composant tibial au cours d'un mouvement de flexion/extension.

2. Dispositif de coupe (1) selon la revendication 1, **caractérisé en ce que** la forme externe de l'organe de positionnement (20) est sensiblement identique au moins à la forme externe des parties distale et postérieure du composant fémoral à implanter.

3. Dispositif de coupe (1) selon l'une des revendications 1 ou 2, **caractérisé en ce que** la forme externe de l'organe de positionnement (20) présente des caractéristiques formelles et dimensionnelles sensiblement identiques à celles qui sont communes aux formes externes des différentes tailles du composant fémoral pouvant être utilisées.

4. Dispositif de coupe (1) selon l'une quelconque des revendications 1 à 3, **caractérisé en ce que** l'organe de positionnement (20) est monté mobile en translation axiale par rapport aux moyens de centrage (10), suivant une direction parallèle à l'axe médullaire de l'os.

5. Dispositif de coupe (1) selon la revendication 4, **caractérisé en ce qu'**il comporte des moyens de blocage (50) aptes à immobiliser, en translation axiale, l'organe de positionnement (20) par rapport aux moyens de centrage (10).

6. Dispositif de coupe (1) selon la revendication 5, **caractérisé en ce que** les moyens de blocage (50) sont réglables en longueur.

7. Dispositif de coupe (1) selon la revendication 6, **caractérisé en ce que** le réglage des moyens de blocage (50) s'effectue par pas de longueur déterminée.

8. Dispositif de coupe (1) selon l'une quelconque des revendications 5 à 7, **caractérisé en ce que** les moyens de blocage (50) sont montés pivotants par rapport audit dispositif de coupe (1), autour d'une direction sensiblement perpendiculaire à l'axe médullaire de l'os.

9. Dispositif de coupe (1) selon l'une quelconque des revendications 5 à 8, **caractérisé en ce que** les moyens de blocage (50) comportent une crémaillère pivotante (51) avec laquelle coopère par blocage un organe de verrouillage mobile (52) disposé dans une tête d'ancrage (53) apte à être solidarisée à l'os, la crémaillère étant solidaire d'au moins un élément dudit dispositif de coupe (1).

10. Dispositif de coupe (1) selon l'une quelconque des revendications 5 à 9, **caractérisé en ce que** les moyens de blocage (50) sont amovibles.

11. Dispositif de coupe (1) selon l'une quelconque des revendications 5 à 10, **caractérisé en ce que** les moyens de blocage (50) sont solidaires de moyens de coupe distale (80).

12. Dispositif de coupe (1) selon l'une quelconque des revendications 1 à 11, **caractérisé en ce que** l'organe de positionnement (20) est monté mobile en rotation axiale par rapport aux moyens de centrage (10), autour d'une direction parallèle à l'axe médullaire de l'os.

13. Dispositif de coupe (1) selon la revendication 12, **caractérisé en ce qu'**il comporte des moyens de visée (70) aptes à déterminer la position en rotation axiale de l'organe de positionnement (20) par rapport aux moyens de centrage (10).

14. Dispositif de coupe (1) selon la revendication 13, **caractérisé en ce que** les moyens de visée (70) comportent une poignée (71) sur laquelle est montée coulissante une tige de contrôle (74) destinée à être pointée sur un repère anatomique.

15. Dispositif de coupe (1) selon l'une des revendications 13 ou 14, **caractérisé en ce que** les moyens de visées (70) sont amovibles.

16. Dispositif de coupe (1) selon l'une quelconque des revendications 1 à 15, **caractérisé en ce que** l'organe de positionnement (20) est monté mobile en translation transversale par rapport aux moyens de centrage (10), suivant une direction orthogonale à l'axe médullaire de l'os.

17. Dispositif de coupe (1) selon la revendication 16, **caractérisé en ce qu'**il comporte des moyens de réglage (24) aptes à déplacer puis à immobiliser, en translation transversale, l'organe de positionnement (20) par rapport aux moyens de centrage (10).

18. Dispositif de coupe (1) selon l'une des revendications 16 ou 17, **caractérisé en ce qu'**il comporte des moyens de calibrage (40) aptes à déterminer la position en translation transversale de l'organe de positionnement (20) par rapport aux moyens de centrage (10), en fonction de la taille du composant fémoral à implanter.

19. Dispositif de coupe (1) selon la revendication 18, **caractérisé en ce que** les moyens de calibrage (40) sont amovibles.

20. Dispositif de coupe (1) selon l'une quelconque des revendications 1 à 19, **caractérisé en ce que** l'organe de positionnement (20) est monté mobile en rotation transversale par rapport aux moyens de centrage (10), autour d'une direction orthogonale à l'axe médullaire de l'os.

21. Dispositif de coupe (1) selon la revendication 20, **caractérisé en ce qu'**il comporte des moyens d'indexage (28) aptes à immobiliser, en rotation transversale, l'organe de positionnement (20) par rapport aux moyens de centrage (10) dans une position relative donnée qui est fonction, d'une part, de la nature droite ou gauche du genou, et d'autre part, de l'inclinaison desdits moyens de centrage (10) par rapport à l'axe mécanique du fémur.

22. Dispositif de coupe (1) selon l'une quelconque des revendications 1 à 21, **caractérisé en ce que** les moyens de centrage (10) comportent un organe intramédullaire destiné à être implanté à l'intérieur du fémur et une tige de guidage (11) apte à coopérer par coulissement avec cet organe intramédullaire.

23. Dispositif de coupe (1) selon la revendication 22, **caractérisé en ce que** l'organe intramédullaire est constitué par une tige filetée (12) formant tire-fond.

24. Dispositif de coupe (1) selon la revendication 22, **caractérisé en ce que** l'organe intramédullaire comporte une tige pourvue à ses extrémités d'un centreur et d'un cône de blocage.

25. Dispositif de coupe (1) selon l'une quelconque des revendications 1 à 24, **caractérisé en ce que** les moyens de coupe de la partie distale de l'os comportent des moyens de coupe distale (80) dotés d'au moins un plan de coupe.

26. Dispositif de coupe (1) selon la revendication 25, **caractérisé en ce que** les moyens de coupe distale (80) sont dotés de plusieurs plans de coupe et les intervalles existant entre les différents plans de coupe distale correspondent aux épaisseurs des différentes cales de comblement disponibles pour compenser les pertes osseuses en parties distales ; lesdites cales venant se solidariser sur les surfaces internes distales du composant fémoral à implanter.

27. Dispositif de coupe (1) selon l'une des revendications 25 ou 26, **caractérisé en ce que** les moyens de coupe distale (80) sont constitués par un gabarit amovible (81) apte à être monté de manière réversible sur l'organe de positionnement (20).

28. Dispositif de coupe (1) selon l'une quelconque des revendications 1 à 27, **caractérisé en ce que** les moyens de coupe de la partie distale de l'os comportent des moyens de coupe postérieure (90) dotés d'au moins un plan de coupe.

29. Dispositif de coupe (1) selon la revendication 28, **caractérisé en ce que** les moyens de coupe postérieure (90) sont dotés de plusieurs plans de coupe et les intervalles existant entre les différents plans de coupe postérieure correspondent aux épaisseurs des différentes cales de comblement disponibles pour compenser les pertes osseuses en parties postérieures ; lesdites cales venant se solidariser sur les surfaces internes postérieures du composant fémoral à implanter.

30. Dispositif de coupe (1) selon l'une des revendications 28 ou 29, **caractérisé en ce que** les moyens de coupe postérieure (90) sont constitués par au moins une fente (91) ménagée directement à travers l'organe de positionnement (20).

31. Dispositif de coupe (1) selon l'une quelconque des revendications 1 à 30, **caractérisé en ce que** les moyens de coupe de la partie distale de l'os comportent des moyens de coupe antérieure (100) montés mobiles en translation sur l'organe de positionnement (20), parallèlement à la direction suivant laquelle ledit organe de positionnement (20) est monté mobile en translation transversale par rapport aux moyens de centrage (10).

32. Dispositif de coupe (1) selon la revendication 31, **caractérisé en ce que** les moyens de coupe antérieure (100) sont constitués par un gabarit amovible (101) comportant des moyens de guidage (102) aptes à coopérer par coulissement avec des moyens de guidage (103) de formes sensiblement complémentaires, ménagés sur l'organe de positionnement (20).

## Claims

1. Femoral cutting jig (1) for the fitting of a total knee replacement prosthesis in orthopaedic surgery, the said cutting jig (1) including alignment means (10) for alignment with respect to the distal end of the femur and means (80, 90, 100) for cutting the distal part of the bone, **characterized in that** the cutting means (80, 90, 100) are fastened to a positioning member (20) that is mounted so as to move in displacement in relation to the alignment means (10), at least one part of the positioning member (20) having an external shape that can cooperate, by sliding, with a tibial component implanted in the tibia, whatever the relative position of the said positioning member (20) in relation to the said tibial component during a bending/extension movement.

2. Cutting jig (1) according to Claim 1, **characterized in that** the external shape of the positioning member (20) is substantially identical to at least the external shape of a distal and a posterior part of the femoral component to be implanted.

3. Cutting jig (1) according to either of Claims 1 and 2, **characterized in that** the external shape of the positioning member (20) has form and dimensional characteristics substantially identical to those that are common to the external shapes of the various sizes of the femoral component that are able to be used.

4. Cutting jig (1) according to any one of Claims 1 to 3, **characterized in that** the positioning member (20) is mounted so as to move in axial displacement relative to the alignment means (10) in a direction parallel to the medullary axis of the bone.

5. Cutting jig (1) according to Claim 4, **characterized in that** it includes immobilizing means (50) that can immobilize, with respect to axial displacement, the positioning member (20) relative to the alignment means (10).

6. Cutting jig (1) according to Claim 5, **characterized in that** the immobilizing means (50) are length-adjustable.

7. Cutting jig (1) according to Claim 6, **characterized in that** the immobilizing means (50) are adjusted in steps of predetermined length.

8. Cutting jig (1) according to any one of Claims 5 to 7, **characterized in that** the immobilizing means (50) are mounted so as to pivot relative to the said cutting jig (1) about a direction approximately perpendicular to the medullary axis of the bone.

9. Cutting jig (1) according to any one of Claims 5 to 8, **characterized in that** the immobilizing means (50) include a pivoting rack (51) with which a movable locking member (52), placed in an anchoring head (53) that can be fastened to the bone, cooperates by immobilization, the rack being fastened to at least one element of the said cutting jig (1).

10. Cutting jig (1) according to any one of Claims 5 to 9, **characterized in that** the immobilizing means (50) are removable.

11. Cutting jig (1) according to any one of Claims 5 to 10, **characterized in that** the immobilizing means (50) are fastened to distal cutting means (80).

12. Cutting jig (1) according to any one of Claims 1 to 11, **characterized in that** the positioning member (20) is mounted so as to move in axial rotation relative to the alignment means (10) about a direction parallel to the medullary axis of the bone.

13. Cutting jig (1) according to Claim 12, **characterized in that** it includes sighting means (70) that can determine the position in terms of axial rotation of the positioning member (20) relative to the alignment means (10).

14. Cutting jig (1) according to Claim 13, **characterized in that** the sighting means (70) include a handle (71) on which is mounted, so as to slide, a control rod (74) intended to be trained on an anatomical reference point.

15. Cutting jig (1) according to either of Claims 13 and 14, **characterized in that** the sighting means (70) are removable.

16. Cutting jig (1) according to any one of Claims 1 to 15, **characterized in that** the positioning member (20) is mounted so as to move in transverse displacement relative to the alignment means (10) in a direction orthogonal to the medullary axis of the bone.

17. Cutting jig (1) according to Claim 16, **characterized in that** it includes adjustment means (24) that can move and then immobilize, in terms of transverse displacement, the positioning member (20) relative to the alignment means (10).

18. Cutting jig (1) according to either of Claims 16 and 17, **characterized in that** it includes calibrating means (40) that can determine the position with respect to transverse displacement of the positioning member (20) relative to the alignment means (10) according to the size of the femoral component to be implanted.

19. Cutting jig (1) according to Claim 18, **characterized in that** the calibrating means (40) are removable.

20. Cutting jig (1) according to any one of Claims 1 to 19, **characterized in that** the positioning member (20) is mounted so as to move in transverse rotation relative to the alignment means (10) about a direction orthogonal to the medullary axis of the bone.

21. Cutting jig (1) according to Claim 20, **characterized in that** it includes indexing means (28) that can immobilize, with respect to transverse rotation, the positioning member (20) relative to the alignment means (10) in a given relative position that depends, on the one hand, on the nature - right or left - of the knee and, on the other hand, on the inclination of the said alignment means (10) relative to the mechanical axis of the femur.

22. Cutting jig (1) according to any one of Claims 1 to 21, **characterized in that** the alignment means (10) include an intramedullary member intended to be implanted inside the femur and a guiding rod (11) that can cooperate, by sliding, with this intramedullary member.

23. Cutting jig (1) according to Claim 22, **characterized in that** the intramedullary member is formed by a threaded rod (12) forming a lag screw.

24. Cutting jig (1) according to Claim 22, **characterized in that** the intramedullary member includes a rod provided at its ends with a centring device and with an immobilizing cone.

25. Cutting jig (1) according to any one of Claims 1 to 24, **characterized in that** the means for cutting the distal part of the bone include distal cutting means (80) provided with at least one cutting plane.

26. Cutting jig (1) according to Claim 25, **characterized in that** the distal cutting means (80) are provided with several cutting planes and the gaps that exist between the various distal cutting planes correspond to the thicknesses of the various filling shims available for compensating for the bone losses in the distal parts, the said shims being fastened to the distal internal surfaces of the femoral component to be implanted.

27. Cutting jig (1) according to either of Claims 25 and 26, **characterized in that** the distal cutting means (80) are formed by a removable template (81) that can be mounted reversibly on the positioning member (20).

28. Cutting jig (1) according to any one of Claims 1 to 27, **characterized in that** the means for cutting the distal part of the bone include posterior cutting means (90) provided with at least one cutting plane.

29. Cutting jig (1) according to Claim 28, **characterized in that** the posterior cutting means (90) are provided with several cutting planes and the gaps that exist between the various posterior cutting planes correspond to the thicknesses of the various filling shims available for compensating for the bone losses in the posterior parts, the said shims being fastened to the posterior internal surfaces of the femoral component to be implanted.

30. Cutting jig (1) according to either of Claims 28 and 29, **characterized in that** the posterior cutting means (90) are formed by at least one slot (91) made directly through the positioning member (20).

31. Cutting jig (1) according to any one of Claims 1 to 30, **characterized in that** the means for cutting the distal part of the bone include anterior cutting means (100) mounted so as to move in displacement on the positioning member (20), parallel to the direction along which the said positioning member (20) is mounted so as to move in transverse displacement relative to the alignment means (10).

32. Cutting jig (1) according to Claim 31, **characterized in that** the anterior cutting means (100) are formed by a removable template (101) having guiding means (102) that can cooperate, by sliding, with guiding means (103) of substantially complementary shape, the said guiding means being provided on the positioning member (20).

## Patentansprüche

1. Femurschneidvorrichtung (1) zum Einsetzen einer vollständigen Prothese eines erneuerten Knies in der Knochenchirurgie, wobei die Schneidvorrichtung (1) Zentrierungsmittel (10) am distalen Endabschnitt des Femurs sowie Schneidmittel (80, 90, 100) des distalen Teils des Knochens aufweist, **dadurch gekennzeichnet, daß** die Schneidmittel (80, 90, 100) fest mit einem Positionierungsorgan (20) verbunden sind, das beweglich hinsichtlich einer Verlagerung in Bezug auf die Zentrierungsmittel (10) gehalten ist, wobei zumindest ein Teil des Positionierungsorgans (20) eine äußere Form aufweist, die dazu geeignet ist, über eine Gleitbewegung mit einer tibialen Komponente zusammenzuwirken, die in die Tibia implantiert ist, unabhängig davon, welches die relative Position des genannten Positionierungsorgans (20) in Bezug auf die tibiale Komponente im Verlaufe einer Beuge- bzw. Streckbewegung ist.

2. Schneidvorrichtung (1) nach Anspruch 1, **dadurch gekennzeichnet, daß** die äußere Form des Positionierungsorgans (20) im wesentlichen identisch zumindest mit der äußeren Form der distalen und posterioren Bereiche der zu implantierenden femuralen Komponente ist.

3. Schneidvorrichtung (1) nach einem der Ansprüche 1 oder 2, **dadurch gekennzeichnet, daß** die äußere Form des Positionierungsorgans (20) Merkmale bzgl. der Form und Abmessungen aufweist, die im wesentlichen identisch sind wie die, die den äußeren Formen der unterschiedlichen Größen der femuralen Komponente, die verwendet werden kann, gemeinsam sind.

4. Schneidvorrichtung (1) nach einem der Ansprüche 1 - 3, **dadurch gekennzeichnet, daß** das Positionierungsorgan (20) beweglich hinsichtlich einer axialen Translation in Bezug auf die Zentrierungsmittel (10) gehalten ist, entlang einer Richtung, die parallel zur medullären Achse des Knochens ist.

5. Schneidvorrichtung (1) nach Anspruch 4, **dadurch gekennzeichnet, daß** sie Blockierungsmittel (50) aufweist, die dazu geeignet sind, das Positionierungsorgan (20) in Bezug auf die Zentrierungsmittel (10) hinsichtlich einer axialen Translation festzusetzen.

6. Schneidvorrichtung (1) nach Anspruch 5, **dadurch gekennzeichnet, daß** die Blockierungsmittel (50) in ihrer Länge einstellbar sind.

7. Schneidvorrichtung (1) nach Anspruch 6, **dadurch gekennzeichnet, daß** die Einstellung der Blockierungsmittel (50) über bestimmte Längenschritte erfolgt.

8. Schneidvorrichtung (1) nach einem der Ansprüche 5 - 7, **dadurch gekennzeichnet, daß** die Blockierungsmittel (50) schwenkbar in Bezug auf die Schneidvorrichtung (1) gehalten sind, um eine Richtung, die im wesentlichen senkrecht zu der medullären Achse des Knochens ist.

9. Schneidvorrichtung (1) nach einem der Ansprüche 5 - 8, **dadurch gekennzeichnet, daß** die Blockierungsmittel (50) eine schwenkbare Zahnstange (51) aufweisen, mit der über Blockierung ein bewegliches Verriegelungsorgan (52) zusammenwirkt, das in einem Verankerungskopf (53) angeordnet ist, der dazu bestimmt ist, fest mit den Knochen verbunden zu werden, wobei die Zahnstange zumindest mit einem Element der Schneidvorrichtung (1) fest verbunden ist.

10. Schneidvorrichtung (1) nach einem der Ansprüche 5 - 9, **dadurch gekennzeichnet, daß** die Blockierungsmittel (50) abnehmbar sind.

11. Schneidvorrichtung (1) nach einem der Ansprüche 5 - 10, **dadurch gekennzeichnet, daß** die Blockierungsmittel (50) fest mit distalen Schneidmitteln (80) verbunden sind.

12. Schneidvorrichtung (1) nach einem der Ansprüche 1 - 11, **dadurch gekennzeichnet, daß** das Positionierungsorgan (20) beweglich im Hinblick auf eine axiale Rotation in Bezug auf die Zentrierungsmittel (10) gehalten ist, um eine Richtung, die parallel zur medullären Achse des Knochens ist.

13. Schneidvorrichtung (1) nach Anspruch 12, **dadurch gekennzeichnet, daß** sie Anvisiermittel (70) aufweist, die dazu bestimmt sind, die Position hinsichtlich einer axialen Rotation des Positionierungssorgangs (20) in Bezug auf die Zentrierungsmittel (10) zu bestimmen.

14. Schneidvorrichtung (1) nach Anspruch 13, **dadurch gekennzeichnet, daß** die Anvisiermittel (70) ein Griffstück (71) aufweisen, auf dem schiebebeweglich ein Steuerstift (74) gehalten ist, der dazu bestimmt ist, auf einen anatomischen Bezugspunkt ausgerichtet zu werden.

15. Schneidvorrichtung (1) nach einem der Ansprüche 13 oder 14, **dadurch gekennzeichnet, daß** die Anvisiermittel (70) abnehmbar sind.

16. Schneidvorrichtung (1) nach einem der Ansprüche 1 - 15, **dadurch gekennzeichnet, daß** das Positionierungsorgan (20) beweglich im Hinblick auf eine quer gerichtete Translation in Bezug auf die Zentrierungsmittel (10) gehalten ist, entlang einer Richtung, die senkrecht zur medullären Achse des Knochens ist.

17. Schneidvorrichtung (1) nach Anspruch 16, **dadurch gekennzeichnet, daß** sie Einstellmittel (24) aufweist, die dazu bestimmt sind, das Positionierungsorgan (20) in Bezug auf die Zentrierungsmittel (10) im Hinblick auf eine quer gerichtete Translation zu verlagern und anschließend festzusetzen.

18. Schneidvorrichtung (1) nach einem der Ansprüche 16 oder 17, **dadurch gekennzeichnet, daß** sie Kalibrierungsmittel (40) aufweist, die dazu bestimmt sind, die Position des Positionierungsorgans (20) im Hinblick auf eine quer gerichtete Translation in Bezug auf die Zentrierungsmittel (10) zu bestimmen, in Abhängigkeit von der Größe der zu implantierenden femuralen Komponente.

19. Schneidvorrichtung (1) nach Anspruch 18, **dadurch gekennzeichnet, daß** die Kalibrierungsmittel (40) abnehmbar sind.

20. Schneidvorrichtung (1) nach einem der Ansprüche 1 - 19, **dadurch gekennzeichnet, daß** das Positionierungsorgan (20) beweglich im Hinblick auf eine quer gerichtete Rotation in Bezug auf die Zentrierungsmittel (10) gehalten ist, um eine Richtung, die senkrecht zur medullären Achse des Knochens ist.

21. Schneidvorrichtung (1) nach Anspruch 20, **dadurch gekennzeichnet, daß** sie Indexierungsmittel (28) aufweist, die dazu bestimmt sind, das Positionierungsorgan (20) im Hinblick auf eine quer gerichtete Rotation in Bezug auf die Zentrierungsmittel (10) in einer vorgegebenen relativen Position festzusetzen, die einerseits davon abhängt, ob es sich um das rechte oder linke Knie handelt, und andererseits von der Neigung der Zentrierungsmittel (10) in Bezug auf die mechanische Achse des Femurs.

22. Schneidvorrichtung (1) nach einem der Ansprüche 1 - 21, **dadurch gekennzeichnet, daß** die Zentrierungsmittel (10) ein intramedulläres Organ aufweisen, das dazu bestimmt ist, ins Innere des Femurs implantiert zu werden, und einen Führungsstift (11), der dazu bestimmt ist, über eine Gleitbewegung mit dem genannten intramedullären Organ zusammenzuwirken.

23. Schneidvorrichtung (1) nach Anspruch 22, **dadurch gekennzeichnet, daß** das intramedulläre Organ durch einen mit Gewinde versehenen Stift (12) gebildet ist, der eine Verankerungsschraube bildet.

24. Schneidvorrichtung (1) nach Anspruch 22, **dadurch gekennzeichnet, daß** das intrameulläre Organ einen Stift aufweist, der an seinen Endabschnitten mit einem Zentrierungselement und einem Blockierungskonus versehen ist.

25. Schneidvorrichtung (1) nach einem der Ansprüche 1 - 24, **dadurch gekennzeichnet, daß** die Schneidmittel des distalen Bereichs des Knochens distale Schneidmittel (80) aufweisen, die mit zumindest einer Schneidebene versehen sind.

26. Schneidvorrichtung (1) nach Anspruch 25, **dadurch gekennzeichnet, daß** die distalen Schneidmittel (80) mit mehreren Schneidebenen versehen sind, wobei die Abstände, die zwischen den unterschiedlichen distalen Schneidebenen bestehen, den Dicken der unterschiedlichen Füllstücke entsprechen, die zur Verfügung stehen, um die Knochenverluste an distalen Bereichen auszugleichen; wobei die genannten Füllstücke sich auf den innenliegenden distalen Oberflächen der zu implantierenden femuralen Komponente fest verbinden.

27. Schneidvorrichtung (1) nach einem der Ansprüche 25 oder 26, **dadurch gekennzeichnet, daß** die distalen Schneidmittel (80) durch eine abnehmbare Schablone (81) gebildet sind, die dazu bestimmt ist, in einer umkehrbaren Weise auf dem Positionierungsorgan (20) angebracht zu werden.

28. Schneidvorrichtung (1) nach einem der Ansprüche 1 - 27, **dadurch gekennzeichnet, daß** die Schneidmittel des distalen Bereichs des Knochens posteriore Schneidmittel (90) aufweisen, die mit zumindest einer Schneidebene versehen sind.

29. Schneidvorrichtung (1) nach Anspruch 28, **dadurch gekennzeichnet, daß** die posterioren Schneidmittel (90) mit mehreren Schneidebenen versehen sind, und daß die Abstände, die zwischen den unterschiedlichen posterioren Schneidebenen vorhanden sind, den Dicken der unterschiedlichen Füllstücke entsprechen, die zur Verfügung stehen, um die Knochenverluste in posterioren Bereichen auszugleichen; wobei die genannten Füllstücke sich auf den innenliegenden posterioren Flächen der zu implantierenden femuralen Komponente fest verbinden.

30. Schneidvorrichtung (1) nach einem der Ansprüche 28 oder 29, **dadurch gekennzeichnet, daß** die posterioren Schneidmittel (90) durch zumindest einen Spalt (91) gebildet sind, der unmittelbar quer zu dem Positionierungsorgan (20) angebracht ist.

31. Schneidvorrichtung (1) nach einem der Ansprüche 1 - 30, **dadurch gekennzeichnet, daß** die posterioren Schneidmittel (90) mit mehreren Schneidebenen versehen sind und anteriore Schneidmittel (100) aufweisen, die beweglich im Hinblick auf eine Translation auf dem Positionierungsorgan (20) gehalten sind, parallel zu der Richtung, entlang der das Positionierungsorgan (20) beweglich im Hinblick auf eine quer gerichtete Translation in Bezug auf die Zentrierungsmittel (10) gehalten ist.

32. Schneidvorrichtung (1) nach Anspruch 31, **dadurch gekennzeichnet, daß** die anterioren Schneidmittel (100) durch eine abnehmbare Schablone (101) gebildet sind, die Führungsmittel (102) aufweist, die dazu bestimmt sind, über eine Gleitbewegung mit Führungsmitteln (103) zusammenzuwirken, die eine im wesentlichen komplementäre Form aufweisen und auf dem Positionierungsorgan (20) angeordnet sind.
